# EUROPEAN PATENT APPLICATION

(11) **EP 1 260 500 A1**
(43) Date of publication of application: **27.11.2002**
(21) Application number: 01420115.6
(22) Date of filing: 18.05.2001
(51) Int. Cl.: C07C 319/18, C07C 323/52, C07C 323/12

(54) **Process for the production of a sulphur-containing organic compound**

(71) Applicant: AVENTIS ANIMAL NUTRITION S.A., 92164 Antony Cedex (FR)
(72) Inventor: Gros, Georges, 92160 Antony (FR); Garrait, Michel, 69390 Millery (FR); Rey, Patrick, 34800 Moureze (FR); Taillades, Jacques, 34830 Clapiers (FR)
(74) Representative: Mérigeault, Shona

(57) **Abstract**

A process for the production of an organic sulphur-containing product which comprises reacting a sulphur-containing compound with an unsaturated hydrocarbon in the presence of a boron compound at a temperature of less than or equal to 50°C.

## Description

The present invention relates to a process for the production of sulphur-containing organic compounds by the catalysed reaction between a sulphur-containing compound and an unsaturated hydrocarbon.

A very large number of papers and patents in the last 15-20 years have dealt with the reaction of substances containing the thiol (SH) groups such as thiols, hydrogen sulphide and thio-acids with various unsaturated compounds. The thiylation reaction is one of the most versatile pathways for the formation of carbon-sulphur bonds and involves the addition of compounds containing a labile S-H bond with unsaturated systems such as alkenes, alkynes and carbonyls as shown below:

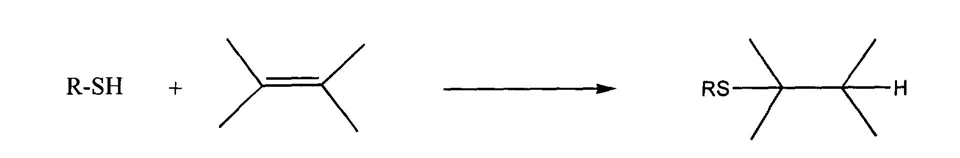

### R = H, alkyl, aryl, R'CO (R' = aryl or alkyl)

The thiylation of unsaturated compounds is also of interest, since these reactions can be regarded as models for certain processes of physiological importance, taking place in living microorganisms, particularly the synthesis of natural mercapto-amino-acids and its derivatives. These reactions provide a convenient method of synthesising various thiols and sulphides with properties of practical interest: regulators for polymerisation processes, vulcanisation accelerators, insecticides, drugs, etc. Some of these syntheses have acquired industrial importance. The thiylation reaction can progress *via* a radical and/or an ionic mechanism. It appears in the literature that the difference in the thiylation reaction mechanism depends upon the nature of the unsaturated substrates. In fact, when the unsaturated substrate is an unsaturated system having an nucleophilic character, the mechanism is preferentially radical. In this case, photoactivation or chemical activation (peroxides, AIBN) is the most efficient method.

A problem associated with such methods is that the reaction time is long and experimental conditions are mostly drastic (temperature > 65°C). Furthermore, the regioselectivity of the addition is weak and purification by fraction distillation is more often necessary. Indeed, when the unsaturated compounds contains electron-withdrawing substituant, the main mechanism is ionic. Trialkylamines or alcoholates are generally used. In the presence of free radical initiators or under photochemical irradiation in homogeneous medium, the thiol reagents add to double bond by a free radical mechanism and the orientation is anti-Markovnikov. In basic medium, the addition occurs by nucleophilic attack of thiolates to olefins by a Michael type mechanism and the orientation is also anti-Markovnikov.

It is known to produce sulphur-containing organic compounds through the reaction of a sulphur-containing compound such , thiols, hydrogen sulphide or thio-acids and an unsaturated hydrocarbon as is evidenced by US Patents No. 2515120, 2411961 and 2828321 and DE 831841. The reactions used in these patent applications are carried out at a temperature of up to 60°C and require an initiator. Furthermore, unwanted by-products are produced which have to be separated at the end of the synthesis route, thereby adding an additional step to the process.

We have found that sulphur-containing organic compounds can be prepared by a simple synthesis route which does not produce undesired by-products and can be carried out under less severe reaction conditions.

Accordingly, the present invention provides a process for the production of organic sulphur-containing product having the general formula (I) 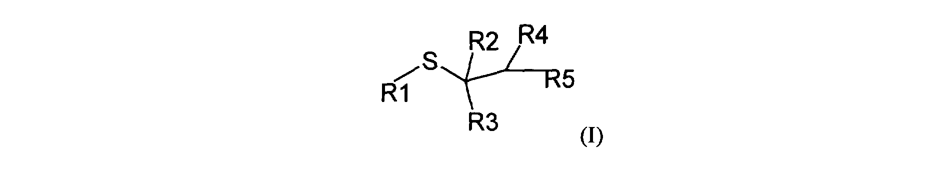 wherein:
R1 is H, alkyl, aryl, arylthio-acid or alkylthio-acid;
R², R³ and R⁴ are each independently hydrogen, alkyl or aryl, and R⁵ is (CH₂)ₙOH; (CH₂)ₙNH₂; (CH₂)ₙCN; R¹⁰NXY, CH(CH₂)ₙOR¹²OR¹³ or 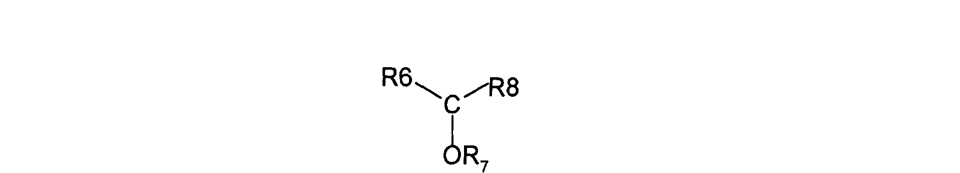
where
R⁶ is H or alkyl;
R⁷ is H, alkyl, aryl or ester;
R⁸ is CN, (CH₂)ₙOH or COOR⁹ where R⁹ is H, alkyl or aryl and n is from 1 to 6;
R¹⁰ is CHCO₂R¹¹ where R¹¹ is hydrogen, alkyl or aryl, and X and Y are independently selected from hydrogen, alkyl, aryl, ester (CH₃CO or PhCO);
R¹² and R¹³ are the same or different and may be hydrogen, alkyl or aryl or the two groups may be linked by (CH₂)ₙ to form a cyclic ring, where n is from 1 to 6;
which process comprises reacting a sulphur-containing compound of general formula (II)

R¹-SH (II)

wherein R¹ is as defined above with an unsaturated hydrocarbon of general formula (III) 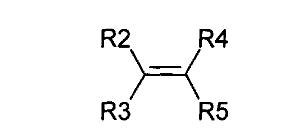 wherein the various symbols are as defined above, in the presence of a boron compound and at a temperature of less than or equal to 50°C.

The process of the present invention provides the advantage over the known prior art in that the reaction conditions are less severe and there is substantially no unwanted by-products.

The process of the present invention involves reacting a sulphur-containing compound of general formula (II) with a unsaturated hydrocarbon. R¹ of compound (II) may be selected from hydrogen, alkyl, aryl or an ester R¹²CO where R¹² is alkyl or aryl. Where R¹ is alkyl, this functional group may be C₁ to C₆ alkyl, preferably C₁ to C₃ alkyl, especially methyl and ethyl. Where R¹ is aryl, this functional group may be phenyl or substituted phenyl, for example halo phenyl, alkoxy phenyl or alkyl phenyl. A particularly preferred compound for use in the present process is where R¹ is methyl or phenyl.

As regards the unsaturated hydrocarbon compound, this compound may be an alkene, an allylic acid, an allylic alcohol, an allylic-alpha-acetoxy nitrile or an alpha amino acid vinyl such as D or L-vinylglycine. In particular, R², R³ and R⁴ of general formula (III) may be the same or may be different and may be hydrogen or alkyl. Where any one of these groups are alkyl, suitable alkyls are C₁ to C₆ alkyl, preferably C₁ to C₃ alkyl, especially methyl. R⁵ is of the formula as given above where R⁶ is H or alkyl; alkyl being as defined above. Preferably R⁶ is hydrogen. R⁷ is H, alkyl, aryl or ester. Where R⁷ is alkyl or aryl, these groups are as defined above. Where R⁷ is ester, this group may be for example CH₃CO, or ether, for example CH₃(CH₂)ₙOCH(CH2)ₙCH₃ or CH₃OCH₂. R⁸ is CN, (CH2)ₙOH or COOR⁹ n is from 0 to 6 and R⁹ is H, alkyl or aryl, these groups being as defined above. Preferably, R⁹ is hydrogen. Examples of preferred unsaturated compounds are 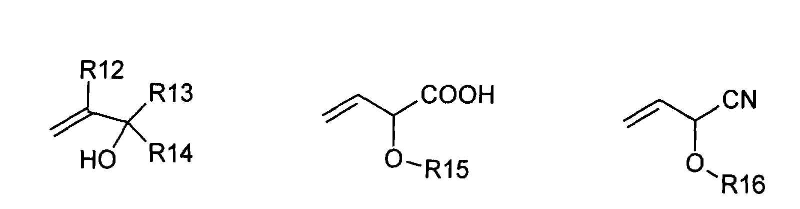 where R12, R13 and R14 are hydrogen or methyl and R15 and R16 are hydrogen, alkyl, aryl, CH₃CO, CH₃CH₂OCHCH₃ or CH₃OCH₂

The process of the present invention is particularly suitable for the preparation of multifunctional sulphides, methionine derivatives and hydroxyanalogue methionine derivatives.

The sulphur-containing compound and the unsaturated hydrocarbon may be reacted in any suitable amount to allow complete reaction. The amount of sulphur-containing compound to unsaturated hydrocarbon may be in a ratio 2 to 1 or 1 to 1, preferably the sulphur-containing compound is present in excess, typically up to 5% excess.

The process of the present invention is carried out in the presence of a boron compound. Suitable boron compounds are alkyl boranes such as methyl, ethyl and propyl. The preferred compound is triethyl borane. The boron compound may be present in an amount of from 0.1 to 30% molar concentration, preferably from 2 to 20%.

The process of the present invention may also be carried out in the presence of ultra violet (uv) irradiation. Although not necessary, it is preferred to use uv irradiation when the unsaturated hydrocarbon contains the CN functional group. The amount of uv light used in such as to enable complete reaction.

The process is carried out at a temperature of less than or equal to 50°C, preferably less that or equal to 20°C. Preferably, the process is operated at a temperature of from - (minus) 20 to + (plus) 20°C.

The process of the present invention will now be illustrated with reference to the following examples:

### Comparative Example A : Synthesis of 4-methylthio-butyric acid using benzoyl peroxide as: CH₃S(CH₂)₃COOH

1.1 mL (0.02 mol) of CH₃SH and 0.024 g (0.1 mmol) of benzoyl peroxide were placed in a closed stirred tank reactor. The reactor contents were stirred and cooled to minus 10°C. 1.72 g (0.02 mol) of vinylacetic acid was introduced into the reactor and the temperature then increased to ambient temperature. The solution was then heated to 80°C. The product obtained gave a 65% yield and was identified by ¹H NMR and mass spectroscopy as 4-methylthio-butyric acid.

### Comparative Example B : Synthesis of 4-methylthio-butyric acid: CH₃S(CH₂)₃COOH using AIBN

The procedure of Comparative Example A was repeated using AIBN instead of benzoyl peroxide. The product obtained gave a 70% yield and was identified by ¹H NMR and mass spectroscopy as 4-methylthio-butyric acid.

### Example 1: Synthesis of 4-methylthio-butyric acid: CH₃S(CH₂)₃COOH

1.72 g (0.02 mol) of vinylacetic acid and 1.1 mL (0.02 mol) of CH₃SH were placed in a closed stirred tank reactor. The mixture was cooled to minus 10°C and stirred for 5 minutes. The vessel was then warmed to ambient temperature. 0.5 mL (0.5 mmol) of molar triethylborane was slowly added. The resulting mixture was stirred for 30 minutes. The gas formed was eliminated and the resulting mixture was washed with 20 mL of water and then extracted with ether (3 x 40 mL). The ether phased dried with sodium sulphate, followed by filtration and evaporation. The product obtained gave a 99% yield and was identified by ¹H NMR and mass spectroscopy as 4-methylthio-butyric acid.

### Example 2: Synthesis of 4-ethylthio-butyric acid : CH₃CH₂S(CH₂)₃COOH

The procedure as for Example 1 was repeated using 1.72 g (0.02 mol) of vinylacetic acid, 1.25 g (0.02 mol) of CH₃CH₂SH and carried out at ambient temperature. 0.5 mL (0.5 mmol) of molar triethylborane was slowly added to the mix. The yield of product obtained was 99% and was identified by ¹H NMR and mass spectroscopy as 4-ethylthio-butyric acid.

### Example 3: Synthesis of 4-hexylthio-butyric acid : n-C₆H₁₃S(CH₂)₃COOH

The procedure as for Example 2 was repeated using 1.72 g (0.02 mol) of vinylacetic acid, 2.36 g (0.02 mol) of CH₃(CH₂)₅SH. 0.5 mL (0.5 mmol) of molar triethylborane was slowly added to the mix. The yield of product obtained was 89% and was identified by ¹H NMR and mass spectroscopy as 4-hexylthio-butyric acid.

### Example 4: Synthesis of 1-methylthio-3-hydroxy-butane : CH₃S(CH₂)₂CH(OH)CH₃

The procedure as for Example 1 was repeated using 1.45 g (0.02 mol) of but-3-en-2-ol and 1.1 mL (0.02 mol) of CH₃SH. 0.5 mL (0.5 mmol) of molar triethylborane was slowly added to the mix. The yield of product obtained was 97% and was identified by ¹H NMR and mass spectroscopy as 1-methylthio-3-hydroxy-butane.

### Example 5: Synthesis of 1-ethylthio-3-hydroxy-butane : CH₃CH₂S(CH₂)₂CH(OH)CH₃

The procedure as for Example 2 was repeated using 1.45 g (0.02 mol) of but-3-en-2-ol and 1.25 g (0.02 mol) of CH₃CH₂SH. 0.5 mL (0.5 mmol) of molar triethylborane was slowly added to the mix. The yield of product obtained was 95% and was identified by ¹H NMR and mass spectroscopy as 1-ethylthio-3-hydroxy-butane.

### Example 6: Synthesis of 1-methylthio-3-hydroxy-3-methyl-butane : CH₃S(CH₂)₂C(CH₃)₂OH

The procedure as for Example 1 was repeated using 1.72 g (0.02 mol) of 2-methylbut-3-en-2-ol and 1.1 mL (0.02 mol) of CH₃SH. 0.5 mL (0.5 mmol) of molar triethylborane was slowly added to the mix. The yield of product obtained was 98% and was identified by ¹H NMR and mass spectroscopy as 1-methylthio-3-hydroxy-3-methyl-butane.

### Example 7: Synthesis of 1-ethylthio-3-hydroxy-3-methyl-butane CH₃CH₂S(CH₂)₂C(CH₃)₂OH

The procedure as for Example 2 was repeated using 1.72 g (0.02 mol) of 2-methylbut-3-en-2-ol and 1.25 g (0.02 mol) of CH₃CH₂SH. 0.5 mL (0.5 mmol) of molar triethylborane was slowly added to the mix. The yield of product obtained was 97% and was identified by ¹H NMR and mass spectroscopy as 1-ethylthio-3-hydroxy-3-methyl-butane.

### Example 8: Synthesis of 1-methylthio-2-methylpropan-3-ol: CH₃SCH₂CH(CH₃)CH₂OH

The procedure as for Example 1 was repeated using 1.45 g (0.02 mol) of 2-methylprop-2-en-1-ol and 1.1 ml (0.02 mol) of CH₃SH. 0.5 mL (0.5 mmol) of molar triethylborane was slowly added to the mix. The yield of product obtained was 99% and was identified by ¹H NMR and mass spectroscopy as 1-methylthio-2-methylpropan-3-ol.

### Example 9: Synthesis of 1-ethylthio-2-methylpropan-3-ol: CH₃CH₂SCH₂CH(CH₃)CH₂OH

The procedure as for Example 2 was repeated using 1.45 g (0.02 mol) of 2-methylprop-2-en-1-ol and 1.25 g (0.02 mol) of CH₃CH₂SH. 0.5mL (0.5 mmol) of molar triethylborane was slowly added to the mix. The yield of product obtained was 98% and was identified by ¹H NMR and mass spectroscopy as 1-ethylthio-2-methylpropan-3 -ol.

### Example 10: Synthesis of 2-hydroxy-4-methylthiobutyronitrile: CH₃S(CH₂)₂CH(OH)CN

3.6 g (0.043 mol) of acrolein cyanhydrin and 4.0 mL (0.072 mol) of CH₃SH were placed in a closed stirred tank reactor. The mixture was cooled to minus 10°C and stirred for 5 minutes. The vessel was then warmed to ambient temperature. 5 mL (5 mmol) of molar triethylborane was slowly added. The resulting mixture was stirred and subjected to UV light until all of the acrolein cyanhydrin was converted. On termination of the reaction, the gas formed was eliminated and the resulting mixture was washed with 30 ml of water and then extracted with ether (3 x 50 ml). The ether phased dried with sodium sulphate, followed by filtration and evaporation. The product obtained gave a 96% yield and was identified by ¹H NMR and mass spectroscopy as 2-hydroxy-4-methylthiobutyronitrile.

### Example 11: Synthesis of 2-hydroxy-4-ethylthiobutyronitrile: CH₃CH₂S(CH₂)₂CH(OH)CN

The procedure as for Example 10 was repeated using 3.06 g (0.037 mol) of acrolein cyanhydrin and 5.0 mL (0.069 mol) of CH₃CH₂SH and carried out at ambient temperature. 5mL (5 mmol) of molar triethylborane was slowly added to the mix. The yield of product obtained was 95% and was identified by ¹H NMR and mass spectroscopy as 2-hydroxy-4-ethylthiobutyronitrile.

### Example 12: Synthesis of 2-acetoxy-4-methylthiobutyronitrile: CH₃S(CH₂)₂CH(CN)OC(O)CH₃

The procedure as for Example 10 was repeated using 2.0 mL (0.02 mol) of 2-acetoxy-but-3-ene nitrile and 1.6 g (0.028 mol) of CH₃SH and carried out at ambient temperature. 4.0 ml (4 mmol) of molar triethylborane was slowly added to the mix. The yield of product obtained was 97% and was identified by ¹H NMR and mass spectroscopy as 2-acetoxy-4-methylthiobutyronitrile.

### Example 13: Synthesis of 2-acetoxy-4-ethylthiobutyronitrile: CH₃CH₂S(CH₂)₂CH(CN)OC(O)CH₃

The procedure as for Example 11 was repeated using 2.0 mL (0.02 mol) of 2-acetoxy-but-3-ene nitrile and 2.0 mL (0.028 mol) of CH₃CH₂SH and carried out at ambient temperature. 4.0 ml (4 mmol) of molar triethylborane was slowly added to the mix. The yield of product obtained was 94% and was identified by ¹H NMR and mass spectroscopy as 2-acetoxy-4-ethylthiobutyronitrile.

### Example 14: Synthesis of 2-(1-ethoxyethoxy)-4-methylthiobutyronitrile: CH₃S(CH₂)₂CH(CN)OCH(CH3)OCH₂CH₃

The procedure as for Example 10 was repeated using 3.2 g (0.02 mol) of 2-(1-ethoxyethoxy)-but-3-ene nitrile and 2.0 ml (0.036 mol) of CH₃SH and carried out at ambient temperature. 2.0 ml (2 mmol) of molar triethylborane was slowly added to the mix. The yield of product obtained was 72% and was identified by ¹H NMR and mass spectroscopy as 2-(1-ethoxyethoxy)-4-methylthiobutyronitrile.

### Example 15: Synthesis of 2-methoxymethoxy-4-methylthiobutyronitrile: CH₃S(CH₂)₂CH(CN)OCH₂OCH₃

The procedure as for Example 10 was repeated using 0.2 g (1.6 mmol) of 2-(methoxyethoxy)-but-3-ene nitrile and 0.2 mL (3.6 mmol) of CH₃SH and carried out at ambient temperature. 0.25 mL (0.25 mmol) of molar triethylborane was slowly added to the mix. The yield of product obtained was 95% and was identified by ¹H NMR and mass spectroscopy as 2-methoxymethoxy-4-methylthiobutyronitrile.

### Example 16: Synthesis of 4-methylthiobutane-1,2-diol: CH₃S(CH₂)₂CH(OH)CH₂OH

2.0 g (0.024 mol) of but-3-ene-1,2-diol and 1.5 mL (0.024 mol) of CH₃SH were placed in a closed stirred tank reactor. The mixture was cooled to minus 10°C and stirred for 5 minutes. The vessel was then warmed to ambient temperature. 0.5 mL (0.5 mmol) of molar triethylborane was slowly added. The gas formed was eliminated and the resulting mixture was washed with 20 mL of water and then extracted with ether (3 x 40 mL). The ether phased dried with sodium sulphate, followed by filtration and evaporation. The product obtained gave a 96% yield and was identified by ¹H NMR and mass spectroscopy as 4-methylthiobutane-1,2-diol.

### Example 17: Synthesis of acid 2-hydroxy-4-methylthiobutanoic acid: CH₃S(CH₂)₂CH(OH)CO₂H

The procedure as for Example 10 was repeated using 6.5 g (0.065 mol) of acid 2-hydroxy-but-3-enol and 4 mL (0.072 mol) of CH₃SH and carried out at ambient temperature. 5 ml (5 mmol) of molar triethylborane was slowly added to the mix. The yield of product obtained was 85% and was identified by ¹H NMR and mass spectroscopy as acid 2-hydroxy-4-methylthiobutanoic acid.

## Claims

1. A process for the production of organic sulphur-containing product having the general formula (1) 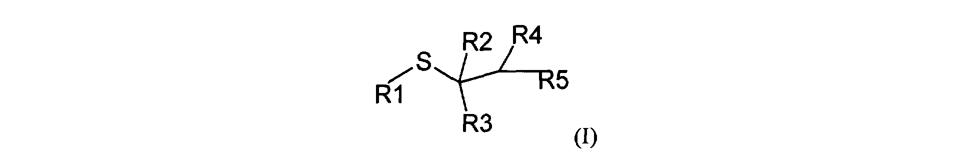 wherein:
R1 is H, alkyl, aryl, aryl thioacid, alkylthio-acid;
R², R³ and R⁴ are each independently hydrogen, alkyl or aryl,
and R⁵ is (CH₂)ₙOH; (CH₂)ₙNH₂; (CH₂)ₙCN_{,} R¹⁰NXY, CH(CH₂)ₙOR¹²OR¹³ or 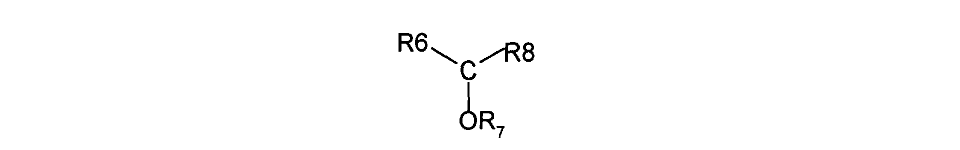
where
R⁶ is H or alkyl,
R⁷ is H, alkyl, aryl or ester;
R⁸ is CN, (CH₂)ₙOH or COOR⁹ where R⁹ is H, alkyl or aryl and n is from 0 to 6, or R¹⁰ is CHCO₂R¹¹ and R¹¹ is hydrogen, alkyl or aryl, and X and Y are independently selected from hydrogen, alkyl, aryl, ester (CH₃CO or PhCO)
R¹² and R¹³ are the same or different and may be hydrogen, alkyl or aryl or the two groups may be linked by (CH₂)ₙ to form a cyclic ring;
n is from 1 to
which process comprises reacting a sulphur-containing compound of general formula (II)
R¹-SH (II)
wherein R¹ is as defined above with an unsaturated hydrocarbon of general formula (III) 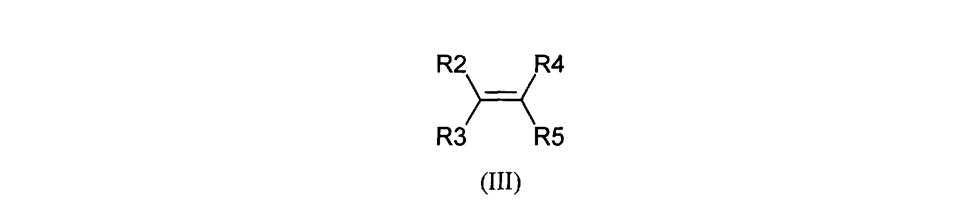 wherein the various symbols are as defined above, in the presence of a boron compound and at a temperature of less than or equal to 50°C.

2. A process as claimed in claim 1 in which the boron compound is an alkyl borane

3. A process as claimed in claim 2 in which the alkyl borane is a C₁ to C₃ alkyl borane.

4. A process as claimed in claim 3 in which the alkyl borane is triethyl borane.

5. A process as claimed in any one of the preceding claims carried out at a temperature of less than or equal to 20°C

6. A process as claimed in any one of the preceding claims carried out at a temperature of from minus 20 to plus 20°C.

7. A process as claimed in any one of the preceding claims wherein compound III is 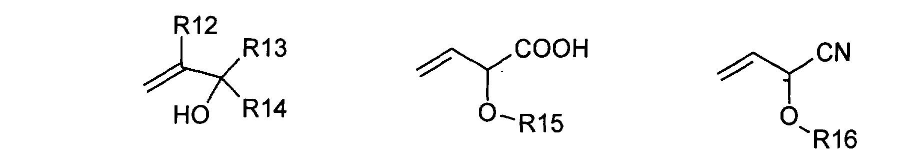 where R12, R13 and R14 are hydrogen or methyl and R15 and R16 are hydrogen, alkyl, aryl, CH₃CO, CH₃CH₂OCH(CH₃) or CH₃OCH₂

8. A process as claimed in any one of the preceding claims wherein R¹ of compound II is alkyl.
